# EUROPEAN PATENT SPECIFICATION

(11) **EP 3 653 180 B1**
(45) Date of publication and mention of the grant of the patent: **04.06.2025**
(21) Application number: 19020582.3
(22) Date of filing: 16.10.2019
(51) Int. Cl.: A61F 5/14

(54) **ORTHOTIC FOR THE CONTROL OF THE FOOT IN INDIVIDUALS WITH MINOR OR MODERATE NEUROMUSCULAR DEFICIENCIES**
ORTHESE ZUR KONTROLLE DES FUSSES BEI PERSONEN MIT GERINGEN ODER MODERATEN NEUROMUSKULÄREN STÖRUNGEN
ORTHÉTIQUE POUR LE CONTRÔLE DU PIED CHEZ LES PERSONNES PRÉSENTANT DES DÉFICIENCES NEUROMUSCULAIRES MINEURES OU MODÉRÉES

(30) Priority: 16.11.2018 IT 201800010412
(43) Date of publication of application: 20.05.2020
(73) Proprietor: Ortopedia Ciaglia Srl, 54100 Massa MS (IT)
(72) Inventor: Bertaccini, Loriana, 54100 Massa (MS) (IT)
(74) Representative: Pietri, Simona

(56) References cited:
- WO-A1-2017/197255
- DE-U1- 202017 101 877
- US-A1- 2001 000 369
- US-A1- 2015 005 687

## Description

### Summary of Invention

Orthotics formed by a shaped slab to be placed between the foot and the insole of the shoe; equipped with medial, lateral and posterior (5) containment wings and with a flexible intermediate area placed in contact with the arch of the foot; said orthotics is designed for patients with minor or moderate neuromuscular deficiency.

### Background Art

Many orthotist apparatuses exist to restore a partial control of the foot in people who partially lost that muscle tone necessary for the control of the foot joints, the majority of these orthosis present complex and noticeable splints or shaped moulds that, running from the bottom of the foot, involve large areas of the leg as well; many of these apparatuses cannot be used along with patient's shoes; some of these apparatuses obtain certain result thanks to a plate, only partially hidden inside the shoe, which extends externally behind the heel to therefore go upwards the rear of the leg.

WO2017/197255A1 discloses an orthotic device for preventing or limiting hyperpronation in a foot, the orthotic device comprising: a heel seat configured for surrounding a lower side of the calcaneus; medial and lateral flanges attached to the heel seat, the medial and lateral flanges configured to extend along sides of the foot along sides of at least portions of the first and fifth metatarsals of the foot, respectively, the heel seat, the medial flanges and the lateral flanges configured to stabilize the foot and limit end range motion in sagittal, frontal and transverse planes; and medial and lateral pads associated with the medial and lateral flanges defining a cutout therebetween, the cutout positioned at least partially on a longitudinal axis of the orthotic device.

US2001/000369A1 discloses an insole having a deep heel cup, and high medial and lateral flanges extending from said heel cup, wherein said heel cup cups the calcaneous of a foot in approximately a neutral degree of varus and said high medial and lateral flanges extend continuously high along the medial and lateral sides of the insole substantially to the first and fifth metatarsal necks of the foot.

Patent N°US9943432.B1 considers a whole range of spring loaded apparatuses mostly made of metallic materials, presumably to be included in orthopedic shoes that apparently have to be tailored once the mechanical contraption is created (Fig. 1). Other inventions, as Patents N°US2015/0005687A1 (fig.2) and N°KR20150007186A (fig.3), envisage a surface placed between the foot and the insole, inside a common shoe, but then these orthotics do not perform the correct foot support functions, neither they collaborate in the flexion of the plantar arch, nor help in the subsequent arch lifting phase. Therefore, at the moment do not exist, for people with minor to moderate neuromuscular deficiencies, orthotics who can be hidden inside the shoe and allow these individuals to correct and hide at the same time their unsteady gait or limping caused by their injury or disease.

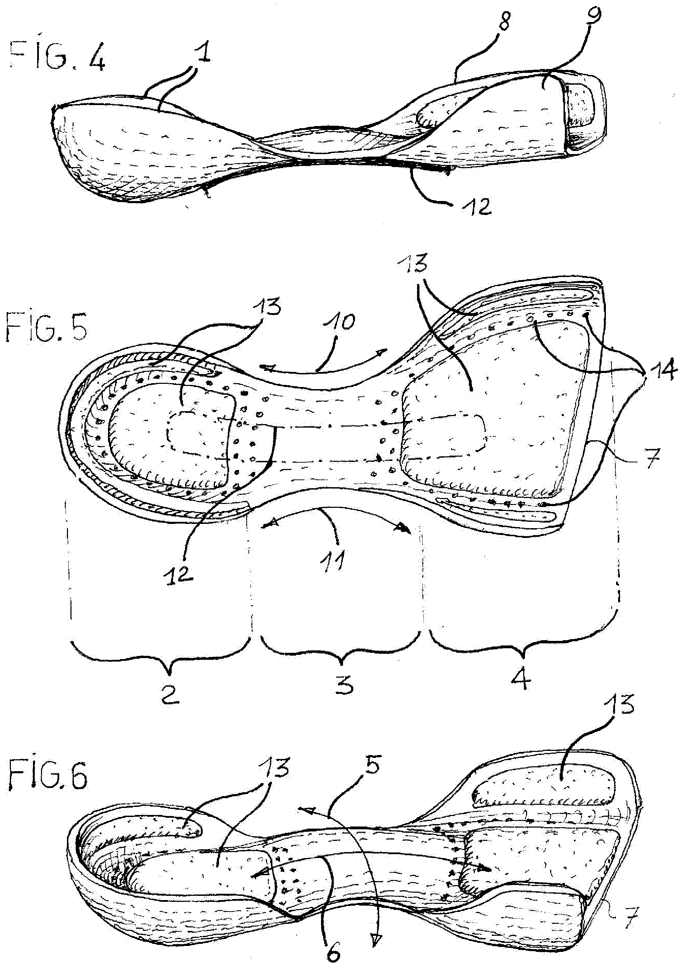

The orthotic object of this patent, comprises a shaped layer to be placed between the foot and the insole within the user's shoe. Said slab (Figs. 4,5,6) is manufactured with semi-rigid and flexible materials and, in some parts of its edges, is equipped with small wings (1,8,9), extending from the horizontal plane of the lamina upwards, that contain and surround heel support area without emerging from the shoe.

In its intermediate area (3), slab is saddle-shape and presents a double orthogonal curvature (5,6); said portion supports and arches the arch of the foot. A third area (4) ends after the metatarsal heads (7) is equipped with two containing wings, one medial (8) and one lateral (9) with function of holding the foot during movement phase.

The slab's portion supporting the arch of the foot instead, is thinned on both sides by two arc millings (10) e (11) the makes it more flexible and facilitate the arch of the foot bending or, during lifting phase, its relaxation.

The orthotic relative rigidity avoid foot torsion caused by any muscle tone defect that nonetheless this insole intends to integrate even if only partly compensate, thus maintaining in such individuals, the exercise of the partial muscle activity they still have.

### Solution to Problem

Capacity of this orthotics of absorbing energy during stance phase and giving it back during lifting phase, together with above mentioned containing function, allows people suffering from partial neuromuscular deficiency to correct their movement and making it more natural.

In many individuals with minor or slight diseases or with pathologies at their early stage indeed, the partial loss of the muscle tone or of the joint alignment causes a wrong plantar stance that visibly underlines such neuromuscular deficit in their gait.

Thanks to the elastic return of the energy due to materials that can be used to produce this orthotic, resistant polymers and elastomers or composite materials, and the possibility to hidden the orthotic inside the shoe, it will be possible for people with partial neuromuscular deficiencies to walk in a natural way.

This orthotic can be placed in every kind of shoes the patient can use, except for flip-flop sandals and slippers, just by simply inserting the orthotic into it.

### Advantageous Effects of Invention

In case of total loss of foot and limb neuromuscular control, complex and noticeable orthoses currently on the market cannot be avoided whereas for minor diseases. The invention in object has the following advantages:
- the invisibility of the object that wholly stays inside an even flat shoe;
- fast use: this orthotics does not need fastener or locking systems and the person can easily change shoes just by inserting the orthotic inside the new ones;
- integration of the sole movement and lateral alignment-torsion of the foot, in which the subject presents neuromuscular deficiency, maintaining his or her muscular capacity in function;
- normalization of the gait avoiding to underline the user's deficit status;
- lightness, cost saving and sustainable materials consumption economy (thanks to the small dimension of the object).

### Description of Embodiments

It is envisaged the adaptability of the product meaning that some variants in size of the orthotic can be tailored and customized to single users for both left and right foot.

Said adjustment will be realized even with small milling intervention to reduce the edges of the flexible section (10) e (11) adapting the orthotic to the weight and anatomy of the specific user.

### Brief Description of Drawings

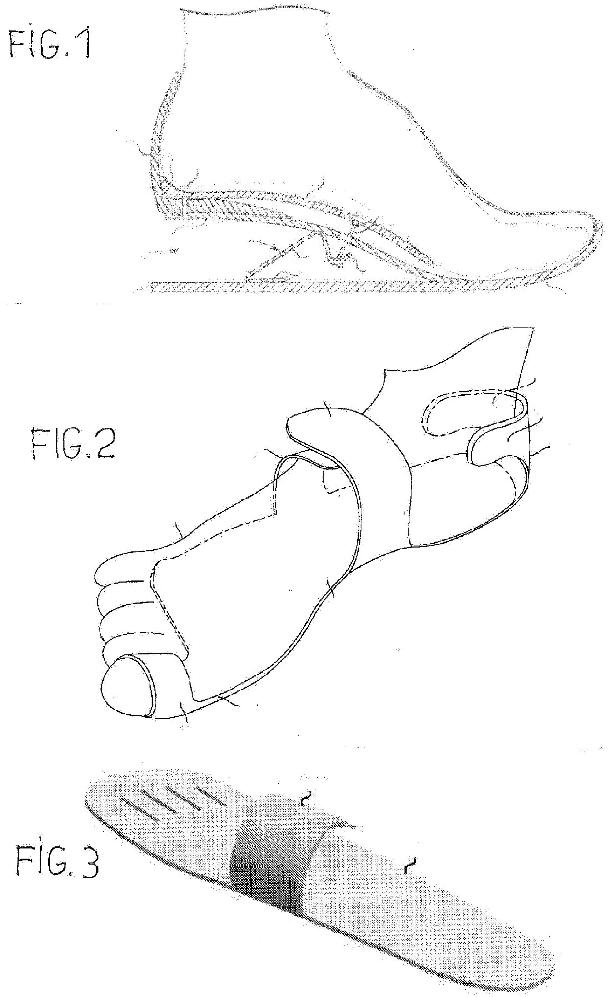

Fig. n°1: Graphics of Patent n° US9943432.B1
Fig. n° 2: Graphics of Patent n° US2015/0005687A1
Fig. n° 3: Graphics of Patent n° KR20150007186A
Fig. n°4 Orthotic lateral view.
Fig. n°5: Orthotic plan view.
Fig. n°6: Orthotic elevation front view.

## Claims

1. An orthotic to be placed between the foot and the insole within the shoes for people with mild and moderate neuromuscular deficiency, wherein said orthotic is configured to be not externally visible when positioned inside a shoe, said orthotic comprises a thin slab divided into three areas: a posterior semirigid area (2) that is adapted, in use, to wrap around from the bottom and contours (1) the heel, an intermediate area (3), and an anterior terminal area (4) being equipped with lateral and medial containing wings (8, 9) and ending, in use, at the anterior line of the medial metatarsal heads (7); **characterised in** said intermediate area (3) being more flexible than said posterior semirigid area (2) and said anterior terminal area (4), and **in that** said said intermediate area (3) being saddle-shaped and supporting the arch of the foot.

2. The orthotic according to claim 1, manufactured with aramid or carbon fiber composite material.

3. The orthotic according to claim 1, consisting of said slab made of polymers thermoformed in a mould.

4. The orthotic according to claim 1, manufactured through injection of polymers in an empty mould, and through the addition of fiberglass or other fibers as functional filler.

5. The orthotic according to one or more of the preceding claims, wherein the edges of the slab are partially or wholly contoured by reinforcing or finishing elements.

6. The orthotic according to one or more of the preceding claims, where on its surfaces reinforcing elements (12) or layers of a material (13) capable of stabilizing and providing a soft contact with the foot are applied.

7. The orthotic according to one or more of the preceding claims, wherein the slab presents micro-holes (14) drilled through.

8. The orthotic according to one or more of the preceding claims, wherein on the basis of the user's weight the width of a middle section of the intermediate area (3) can be reduced through the notching or the milling of its edges (10) and (11).

## Patentansprüche

1. Orthese, um zwischen dem Fuß und der Innensohle innerhalb der Schuhe für Personen mit leichter bis mittelschwerer neuromuskulärer Beeinträchtigung platziert zu werden, wobei die Orthese konfiguriert ist, um, wenn sie in einem Schuh positioniert ist, von außen nicht sichtbar zu sein, wobei die Orthese eine dünne Platte umfasst, die in drei Bereiche unterteilt ist: einen hinteren halbstarren Bereich (2), der angepasst ist, um sich bei Gebrauch von unten her um die Ferse zu legen und diese zu konturieren (1), einen Zwischenbereich (3) und einen vorderen Endbereich (4), der mit einem lateralen und einem medialen Halteflügel (8, 9) ausgestattet ist und bei Gebrauch an der Vorderseite der medialen Mittelfußköpfe (7) endet; **dadurch gekennzeichnet, dass** der Zwischenbereich (3) flexibler als der hintere halbstarre Bereich (2) und der vordere Endbereich (4) ist und dass der Zwischenbereich (3) sattelförmig ist und den Fußrücken stützt.

2. Orthese nach Anspruch 1, hergestellt aus Aramid- oder Kohlefaserverbundwerkstoff.

3. Orthese nach Anspruch 1, bestehend aus der Platte aus in einer Form thermogeformten Polymeren.

4. Orthese nach Anspruch 1, hergestellt durch Einspritzen von Polymeren in eine leere Form und durch die Zugabe von Glasfasern oder anderen Fasern als funktionellem Füllstoff.

5. Orthese nach einem oder mehreren der vorstehenden Ansprüche, wobei die Kanten der Platte durch Verstärkungs- oder Abschlusselemente teilweise oder vollständig konturiert sind.

6. Orthese nach einem oder mehreren der vorstehenden Ansprüche, wobei auf ihren Oberflächen Verstärkungselemente (12) oder Schichten aus einem Material (13) aufgebracht sind, das in der Lage ist, zu stabilisieren und einen weichen Kontakt mit dem Fuß bereitzustellen.

7. Orthese nach einem oder mehreren der vorstehenden Ansprüche, wobei die Platte durchgebohrte Mikrolöcher (14) aufzeigt.

8. Orthese nach einem oder mehreren der vorstehenden Ansprüche, wobei die Breite eines mittleren Abschnitts des Zwischenbereichs (3) durch die Einkerbung oder die Fräsung seiner Kanten (10) und (11) auf Basis des Benutzergewichts reduziert werden kann.

## Revendications

1. Orthèse destinée à être placée entre le pied et la semelle intérieure dans des chaussures pour des personnes souffrant d'une insuffisance neuro-musculaire légère à modérée, dans laquelle ladite orthèse est conçue pour ne pas être visible de l'extérieur lorsqu'elle est positionnée à l'intérieur d'une chaussure, ladite orthèse comprend une mince plaque divisée en trois zones : une zone postérieure semi-rigide (2) qui est adaptée, en cours d'utilisation, pour envelopper à partir du fond et des contours (1) le talon, une zone intermédiaire (3) et une zone antérieure terminale (4) étant équipée d'ailettes de contention latérale et médiale (8, 9) et se terminant, en cours d'utilisation, au niveau de la ligne antérieure des têtes métatarsiennes médiales (7) ; **caractérisée en ce que** ladite zone intermédiaire (3) est plus flexible que ladite zone postérieure semi-rigide (2) et ladite zone antérieure terminale (4), et **en ce que** ladite zone intermédiaire (3) est en forme de selle et supporte la voûte plantaire du pied.

2. Orthèse selon la revendication 1, fabriquée avec un matériau composite de fibres d'aramide ou de carbone.

3. Orthèse selon la revendication 1, constituée par ladite plaque fabriquée en polymères thermoformés dans un moule.

4. Orthèse selon la revendication 1, fabriquée par injection de polymères dans un moule vide, et par le biais de l'addition de fibres de verre ou d'autres fibres en tant que charge fonctionnelle.

5. Orthèse selon l'une ou plusieurs des revendications précédentes, dans laquelle les bords de la plaque sont partiellement ou entièrement entourés par des éléments de renfort ou de finition.

6. Orthèse selon l'une ou plusieurs des revendications précédentes, où sur ses surfaces des éléments de renfort (12) ou des couches d'un matériau (13) capables de stabiliser et de fournir un contact doux avec le pied sont appliqués.

7. Orthèse selon l'une ou plusieurs des revendications précédentes, dans laquelle la plaque présente des micro-trous (14) percés.

8. Orthèse selon l'une ou plusieurs des revendications précédentes, dans laquelle en fonction du poids de l'utilisateur la largeur d'une section médiane de la zone intermédiaire (3) peut être réduite par encochage ou fraisage de ses bords (10) et (11).
